# FASCICULE DE BREVET EUROPEEN

(11) **EP 2 195 283 B1**
(45) Date de publication et mention de la délivrance du brevet: **14.09.2011**
(21) Numéro de dépôt: 08805046.3
(22) Date de dépôt: 03.10.2008
(51) Int. Cl.: C07C 209/84

(54) **PROCEDE DE PURIFICATION D'HEXAMETHYLENE DIAMINE**
VERFAHREN ZUR AUFREINIGUNG VON HEXAMETHYLENDIAMIN
METHOD FOR PURIFYING HEXAMETHYLENE DIAMINE

(30) Priorité: 11.10.2007 FR 0707134
(43) Date de publication de la demande: 16.06.2010
(73) Titulaire: Rhodia Opérations, 93306 Aubervilliers (FR)
(72) Inventeur: BROGLIO, Maria, Ignez, CEP 13023-200 Campinas - São Paulo (BR); AMOROS, Daniel, F-69200 Venissieux (FR); VANNIER, Jean, F-69720 Saint Bonnet De Mure (FR); LETOURNEUR, Didier, F-68170 Rixheim (FR)
(74) Mandataire: Chatelan, Florence Anne
(86) Numéro de dépôt international: PCT/EP2008/063288
(87) Numéro de publication internationale: WO 2009/047219

(56) Documents cités:
- FR-A- 2 892 118
- US-A- 3 017 331
- US-A- 5 192 399
- US-A1- 2003 023 083

## Description

La présente invention concerne un procédé de purification d'hexaméthylène diamine.

Elle concerne plus particulièrement un procédé de purification d'hexaméthylène diamine par séparation de la tétrahydroazépine, ou plus généralement des imines présentes dans l'hexaméthylène diamine.

L'hexaméthylène diamine est un grand intermédiaire chimique utilisée notamment comme monomère pour la fabrication des polymères tels que les polyamides ou comme intermédiaire de synthèse de composés isocyanates.

L'hexaméthylène diamine est obtenue par hydrogénation de l'adiponitrile en présence de catalyseurs comprenant des éléments métalliques tels que nickel, cobalt, fer, rhodium ou ruthénium.

Au cours du procédé d'hydrogénation de l'adiponitrile, des sous-produits sont formés, plus particulièrement des imines et encore plus particulièrement la tétrahydroazépine ( ci-après dénommée par le sigle THA) de formule suivante :

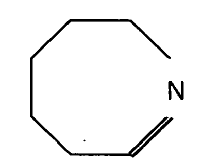

Ce composé doit être séparé de l'hexaméthylène diamine car il peut provoquer une coloration ou des branchements dans les polyamides tels que le PA66 obtenus avec une hexaméthylène diamine contenant de la tétrahydroazépine.

De nombreux procédés ont été proposés pour éliminer ou séparer la THA. Ces procédés comprennent généralement l'addition d'un composé basique pour favoriser la réaction de la THA avec l'hexaméthylène diamine pour former un nouveau composé lourd ou oligomère généralement séparable de l'hexaméthylène diamine.

Ainsi le brevet US 5 192 399 décrit un procédé de distillation d'un mélange hexaméthylène diamine (HMD), aminocapronitrile (ACN) comprenant de la THA. Ce mélange est distillé dans une colonne à distiller en présence d'une solution d'un composé basique. L'aminocapronitrile et l'hexaméthylène diamine sont recueillis en tête de colonne tandis que les oligomères contenant la THA ou composés lourds sont récupérés dans la fraction de fond de la colonne. L'utilisation d'un composé basique génère des effluents salins qui ne peuvent être rejetés sans traitement.

Le document US 2003/023083 décrit un procédé de séparation par distillation d'une azépine telle que la THA, d'une amine telle que l'HMD, en mettant en oeuvre une température de pied de colonne d'au plus 120°C.

Il est donc important de proposer un procédé de séparation de la THA permettant de récupérer l'hexaméthylène diamine contenant une très faible concentration de THA et avec notamment une génération très faibles d'effluents à rejeter.

Un des buts de la présente invention est de proposer un procédé simple de séparation de l'hexaméthylène diamine et de la tétrahydroazépine avec une récupération d'HMD contenant une très faible concentration de THA et une perte en HMD minimalisée.

A cet effet, l'invention propose un procédé de purification d'hexaméthylène diamine par séparation de la tétrahydroazépine consistant à distiller dans une colonne à distiller, le mélange contenant l'hexaméthylène diamine et la tétrahydroazépine. L'invention se caractérise en ce que le temps de séjour de l'hexaméthylène diamine dans la colonne est compris entre une minute et vingt minutes, de préférence entre une minute et quinze minutes.

Par temps de séjour dans une colonne à distiller, on entend le rapport entre le volume de liquide retenu dans la colonne et le débit de liquide circulant à l'intérieur de la colonne. Dans le cas de l'invention, la partie de la colonne à distiller à considérer pour le calcul du temps de séjour est la partie des internes de la colonne situés au-dessus du point d'alimentation de l'hexaméthylène diamine à purifier. Ainsi, les temps de séjour dans le fond de la colonne, dans le bouilleur et dans le condenseur de la colonne ne sont pas à prendre en compte. De même, le temps de séjour dans les internes contenus dans la partie de la colonne située au-dessous du point d'alimentation de l'hexaméthylène diamine n'est pas à prendre en compte. En d'autres termes, pour le calcul du temps de séjour, le terme colonne à distiller utilisé dans le présent texte correspond à la partie internes présents dans la partie de la colonne située au-dessus du point d'alimentation de l'hexaméthylène diamine.
Par interne contenu dans une colonne à distiller, on entend les éléments ou dispositifs disposés dans la colonne pour favoriser et réaliser l'échange liquide/vapeur. A titre d'exemples d'internes on peut citer les garnissages structurés ou non, les plateaux à cloche, à clapets ou analogues. Les internes constitués par un garnissage structuré sont préférés dans le procédé de l'invention.

Le volume de liquide retenu dans la colonne est fonction du type d'internes, de colonne, des dimensions et des conditions opératoires (débits gaz et liquide dans la colonne). Ainsi, pour une colonne à garnissage, le volume de liquide est calculé à partir du volume de garnissage et du taux de rétention du garnissage (volume de liquide par unité de volume de garnissage). Le volume de garnissage est, selon l'invention, le volume contenu dans la partie de la colonne située au-dessus du point d'alimentation de l'hexaméthylène diamine. Généralement , le taux de rétention d'un type de garnissage est indiqué par le fabricant ou fournisseur du garnissage et est une caractéristique de construction de ce matériau et des conditions opératoires de la distillation.

Pour une colonne à plateaux, le volume de liquide retenu correspond au volume de liquide présent sur chaque plateau multiplié par le nombre de plateaux réels

Selon une autre caractéristique de l'invention, le procédé est mis en oeuvre dans des colonnes à distiller comprenant entre 10 et 50 plateaux théoriques, de préférence entre 15 et 35. Ce nombre de plateaux correspond à la partie de la colonne située au-dessus du point d'alimentation de l'hexaméthylène diamine.

Le procédé de l'invention peut être mis en oeuvre dans tout type de colonnes à distiller permettant d'obtenir des temps de séjour tels que décrits précédemment.

Avantageusement, les colonnes à distiller de l'invention sont celles présentant une faible perte de charge. On peut citer comme type de colonnes à distiller convenables, les colonnes à garnissage structuré, les colonnes à distiller à film mince.

Selon une autre caractéristique de l'invention, le procédé est mis en oeuvre dans des colonnes à distiller fonctionnant sous une pression opératoire en tête de colonne comprise entre 10 et 300 mbar. La température de fond de colonne est avantageusement comprise entre 120°C et 170°C.

Le mélange à distiller est, de préférence, alimenté au niveau d'un plateau intermédiaire de la colonne, l'hexaméthylène diamine purifiée récupérée comme fraction de tête ou comme fraction intermédiaire à un niveau proche de la tête de colonne comprend, selon le procédé de l'invention, moins de 8 mol de THA par million de mol de HMD, avantageusement moins de 4 mol de THA par million de mol de HMD.

La concentration en THA ou en impuretés réductibles avec une électrode au mercure dans l'hexaméthylène diamine est déterminée par dosage par polarographie impulsionnelle. Ces impuretés présentent une vague polarographique de réduction à un voltage de -1.55 V/Ag-AgCl +/-0.05V et sont exprimées en mmol d'isobutanal par tonne d'HMD (mmol iB/t) ou en mol de THA par million de moles d'HMD (mpM).

D'autres détails et avantages de l'invention apparaîtront plus clairement au vu des exemples donnés ci-dessous uniquement à titre indicatif.

**Exemple 1 :**

Pour purifier de l'hexaméthylène diamine contenant 120 mol de THA par million de mol de HMD, on utilise une colonne à distiller à garnissage structuré de diamètre 50 mm comprenant 2.6 litres de garnissage tissé avec un taux de rétention de 6%. Cette colonne présente un nombre de plateaux théoriques égal à 15. L'alimentation de l'hexaméthylène diamine est située en-dessous de la partie inférieure du garnissage.

La distillation est réalisée avec une pression de tête de 300 mbar, une température de pied de colonne de 160°C et un débit d'alimentation de HMD de 2.5 kg/h (3.3 l/h). Le débit de liquide de la colonne est déterminé par calcul avec les outils de modélisation utilisés classiquement pour le calcul des colonnes à distiller (logiciel ASPEN^{®}). Le calcul montre que ce débit est voisin du débit d'alimentation dans le cas du présent exemple.

Le temps de séjour de l'hexaméthylène diamine est de 3 minutes.

L'hexaméthylène diamine recueilli en tête de colonne contient 2.3 mol de THA par million de mol de HMD.

**Exemple 2 :**

L'essai 1 est répété à l'exception que le garnissage utilisé est un garnissage tollé avec un taux de rétention de 5% et un volume de garnissage de 7 litres. Le nombre de plateaux théoriques est de 20.

Le temps de séjour de HMD est de 6 mn. L'hexaméthylène diamine récupérée en tête de colonne contient 2.9 mol de THA par million de mol de HMD.

**Exemple 3 :**

L'exemple 1 est répété. Le volume de garnissage est de 3,6 l est le nombre de plateaux théoriques est égal à 20.

Le temps de séjour de HMD dans la colonne est de 4 mn. L'hexaméthylène diamine recueillie en tête de colonne contient 3.9 mol de THA par million de mol de HMD.

**Exemple comparatif :**

Pour purifier de l'hexaméthylène diamine contenant 120 mol de THA par million de mole de HMD. on utilise une colonne à distiller à plateaux de diamètre 50 mm, comprenant 30 plateaux réels avec une rétention de 12 ml par plateau. Cette colonne présente un nombre de plateaux théoriques égal à 17. L'alimentation de l'hexaméthylène diamine est située en-dessous du plateau inférieur de la colonne.

La distillation est réalisée avec une pression de tête de 260 mbar, une température de pied de colonne de 160°C et un débit d'alimentation de HMD de 0.5 kg/h (0.66 l/h). Le débit de liquide de la colonne est déterminé par calcul avec les outils de modélisation utilisés classiquement pour le calcul des colonnes à distiller. Le calcul montre qu'il est voisin du débit d'alimentation dans le cas du présent exemple.

Le temps de séjour de l'hexaméthylène diamine est de 33 minutes.

L'hexaméthylène diamine recueilli en tête de colonne contient 16.4 mol de THA par million de mol de HMD.

## Revendications

1. Procédé de purification d'hexaméthylène diamine (HMD) par séparation de la tétrahydroazépine (THA) consistant à distiller, dans une colonne à distiller, le mélange contenant l'hexaméthylène diamine et la tétrahydroazépine **caractérisé en ce que** le temps de séjour du mélange dans la colonne à distiller est compris entre une minute et vingt minutes.

2. Procédé selon la revendication 1, **caractérisé en ce que** le temps de séjour précité est compris entre une minute et quinze minutes.

3. Procédé selon la revendication 1 ou 2, **caractérisé en ce que** le nombre de plateaux théoriques de la colonne à distiller est compris entre 10 et 50.

4. Procédé selon l'une des revendications précédentes, **caractérisé en ce que** la température de fond de colonne à distiller est comprise entre 120°C et 170°C.

5. Procédé selon l'une des revendications précédentes, **caractérisé en ce que** l'hexaméthylène diamine purifiée contient moins de 8 mol de THA par million de mol de HMD.

6. Procédé selon la revendication 5, **caractérisé en ce que** l'hexaméthylène diamine purifiée contient moins de 4 mol de THA par million de mol de HMD.

7. Procédé selon l'une des revendications précédentes, **caractérisé en ce qu'**il est mis en oeuvre dans une colonne à distiller de type colonne à garnissage structuré.

## Claims

1. Process for the purification of hexamethylenediamine (HMD) by separation from the tetrahydroazepine (THA) which consists in distilling, in a distillation column, the mixture comprising the hexamethylenediamine and the tetrahydroazepine, **characterized in that** the residence time of the mixture in the distillation column is between one minute and twenty minutes.

2. Process according to Claim 1, **characterized in that** the abovementioned residence time is between one minute and fifteen minutes.

3. Process according to Claim 1 or 2, **characterized in that** the number of theoretical plates of the distillation column is between 10 and 50.

4. Process according to one of the preceding claims, **characterized in that** the distillation column bottom temperature is between 120°C and 170°C.

5. Process according to one of the preceding claims, **characterized in that** the purified hexamethylenediamine comprises less than 8 mol of THA per million mol of HMD.

6. Process according to Claim 5, **characterized in that** the purified hexamethylenediamine comprises less than 4 mol of THA per million mol of HMD.

7. Process according to one of the preceding claims, **characterized in that** it is implemented in a distillation column of structured packed column type.

## Patentansprüche

1. Verfahren zur Reinigung von Hexamethylendiamin (HMD) durch Abtrennung von Tetrahydroazepin (THA), bei dem man in einer Destillationskolonne das Gemisch von Hexamethylendiamin und Tetrahydroazepin destilliert, **dadurch gekennzeichnet, daß** die Verweilzeit des Gemischs in der Destillationskolonne zwischen einer Minute und zwanzig Minuten liegt.

2. Verfahren nach Anspruch 1, **dadurch gekennzeichnet, daß** die obige Verweilzeit zwischen einer Minute und fünfzehn Minuten liegt.

3. Verfahren nach Anspruch 1 oder 2, **dadurch gekennzeichnet, daß** die Zahl theoretischer Trennstufen der Destillationskolonne zwischen 10 und 50 liegt.

4. Verfahren nach einem der vorhergehenden Ansprüche, **dadurch gekennzeichnet, daß** die Sumpftemperatur der Destillationskolonne zwischen 120°C und 170°C liegt.

5. Verfahren nach einem der vorhergehenden Ansprüche, **dadurch gekennzeichnet, daß** das gereinigte Hexamethylendiamin weniger als 8 mol THA pro Million mol HMD enthält.

6. Verfahren nach Anspruch 5, **dadurch gekennzeichnet, daß** das gereinigte Hexamethylendiamin weniger als 4 mol THA pro Million mol HMD enthält.

7. Verfahren nach einem der vorhergehenden Ansprüche, **dadurch gekennzeichnet, daß** man es in einer Destillationskolonne vom Packungskolonnentyp durchführt.
